# EUROPEAN PATENT APPLICATION

(11) **EP 3 758 021 A1**
(43) Date of publication of application: **30.12.2020**
(21) Application number: 20182076.8
(22) Date of filing: 24.06.2020
(51) Int. Cl.: G16H 40/67, G16H 20/30, G16H 40/40, A61B 5/00, A61B 5/11, A61H 3/00, A63B 21/00

(54) **EQUIPMENT DATA PROVIDING SYSTEM**

(30) Priority: 28.06.2019 JP 2019121546
(71) Applicant: TOYOTA JIDOSHA KABUSHIKI KAISHA, TOYOTA-SHI, AICHI-KEN 471-8571 (JP)
(72) Inventor: ODASHIMA, Tadashi, Aichi-ken, 471-8571 (JP)
(74) Representative: D Young & Co LLP

(57) **Abstract**

A walking training apparatus according to related art has a problem that a state of walking training undergone outside a medical service provider cannot be acquired. An equipment data providing system according to the present disclosure includes a database (42) configured to manage sensor data (WL) measured by a sensor of equipment (20) from a wearer (11) for walking training, adjustment information indicating an adjustment state of the equipment (20), and the equipment (20) or the wearer (11) in association with each other, and a transmission information management unit (43) configured to transmit information managed in the database (42) in response to an inquiry request from outside to an inquirer who makes the inquiry request.

## Description

### BACKGROUND

The present disclosure relates to an equipment data providing system. In particular, the present disclosure relates to an equipment data providing system that provides data including an equipment usage history of a wearer who wears equipment to a plurality of related parties.

Walking training aimed at restoring and improving a patient's walking function has been performed by making the patient undergo rehabilitation using the equipment that assists him/her to walk with a reduced walking function. Japanese Patent No. 6052234 discloses an example of such a rehabilitation support apparatus and equipment.

The walking training apparatus described in Japanese Patent No. 6052234 is characterized by including a walking assistance apparatus, first pulling means, and second pulling means. The walking assistance apparatus is worn on a user's leg, and assists the user to walk, the first pulling means pulls at least one of the walking assistance apparatus and the user's leg upward and forward, and the second pulling means pulls at least one of the walking assistance apparatus and the user's leg upward and backward.

### SUMMARY

However, in such rehabilitation, training away from a medical service provider, for example, at home, is important in addition to diagnosing the user's recovery at a hospital and training at a rehabilitation facility. In such off-site training, for example, the walking assistance apparatus described in Japanese Patent No. 6052234 is used as equipment. However, the only way to know how a patient uses the equipment is to ask him/her, and thus there is a problem that it is difficult for a medical service provider to know whether there is a problem in the patient's training or in the setting of the equipment, when there is a delay in the patient's recovery level.

The present disclosure has been made to solve such a problem. An object of the present disclosure is to accurately understand a usage state of equipment outside a facility of a medical service provider.

An example aspect of the present disclosure is an equipment data providing system including: a database configured to manage sensor data and adjustment information, and equipment or a wearer in association with each other, the sensor data being measured by a sensor of the equipment worn by the wearer for walking training, and the adjustment information indicating an adjustment state of the equipment; and a transmission information management unit configured to transmit information managed in the database in response to an inquiry request from outside to an inquirer who makes the inquiry request.

With the equipment data providing system according to the present disclosure, information acquired in walking training of the wearer undergone in a remote place can be appropriately referred to via a database.

According to the present disclosure, it is possible to accurately understand information about equipment related to walking training undergone in a remote place away from a medical service provider and to improve an accuracy of a diagnosis on walking training.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a schematic diagram of an equipment user support system according to a first embodiment;
Fig. 2 is a diagram for explaining an overview of an example of equipment according to the first embodiment;
Fig. 3 is a block diagram showing an internal configuration of the equipment according to the first embodiment;
Fig. 4 is a block diagram of an equipment data providing system according to the first embodiment; and
Fig. 5 is a sequence diagram showing a usage mode of the equipment user support system according to the first embodiment.

### DESCRIPTION OF EMBODIMENTS

### [First embodiment]

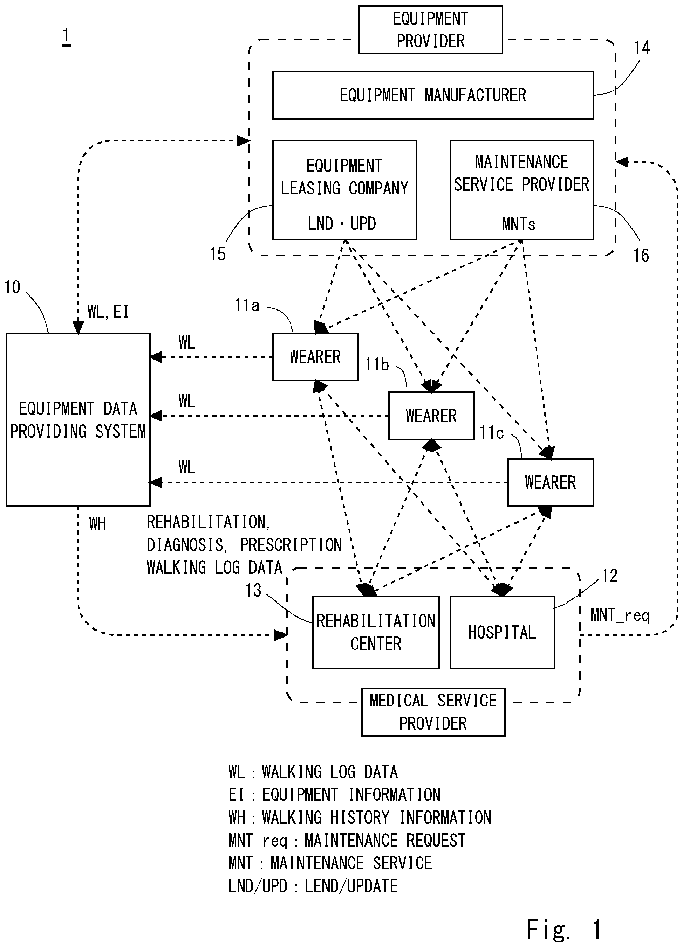

First, Fig. 1 is a schematic diagram of an equipment user support system 1 according to a first embodiment. The schematic diagram of Fig. 1 shows a relationship between wearers who use walking training apparatuses (e.g., equipment) and apparatuses, vendors, and a medical service provider that support the wearers. In the present application, a system including people and apparatuses related to the support of the wearers is referred to as an equipment user support system 1.

As shown in Fig. 1, in the equipment user support system 1 according to the first embodiment, wearers 11a to 11c each wearing the equipment are shown. The wearers 11a to 11c undergo walking training using the equipment in a remote place (e.g., at home) away from the medical service provider. The equipment includes various sensors. When the wearer undergoes walking training, the sensors transmit sensor data indicating the wearer's progress in the walking training to the equipment data providing system 10.

The equipment data providing system 10 stores the sensor data obtained from the equipment worn by the wearers 11a to 11c in a database. Further, adjustment information indicating an adjustment state of the equipment is provided to the equipment data providing system 10 from an equipment provider. Then, the equipment data providing system 10 manages the sensor data and the adjustment information of the equipment in association with the equipment or the wearer. Further, the equipment data providing system 10 includes a transmission information management unit that transmits, in response to an inquiry request from outside, information managed in the database to an inquirer who made the inquiry request. Details of the equipment data providing system 10 including the database and the transmission information management unit will be described later.

Further, in the equipment user support system 1, the wearers 11a to 11c visit a medical service provider including a hospital 12 and a rehabilitation center 13. Then, in the medical service provider, a prescription including a request for adjusting the equipment based on the diagnosis, rehabilitation, and the diagnosis result is given to the wearers 11a to 11c. In a medical service provider, diagnosis and rehabilitation based on walking log data acquired from the equipment are performed. The medical service provider acquires the walking log data from the equipment itself and also refers to walking history information, which is the history information of the walking log data acquired from the training undergone by the wearer at a place away from the medical service provider, from the equipment data providing system 10.

Further, the medical service provider creates setting information of the equipment as a prescription, and passes it to the equipment provider as a maintenance request including the setting information of the equipment. As the equipment provider, an equipment manufacturer 14 that manufactures equipment, an equipment leasing company 15 that purchases the equipment from the equipment manufacturer 14, lends the purchased equipment to the wearers, and provides renewal services to the wearers, and a maintenance service provider 16 that adjusts setting values of the equipment.

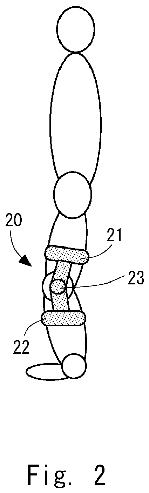

Here, an example of the equipment worn by the wearers 11a to 11c will be described. Fig. 2 is a diagram for explaining an overview of an example of the equipment according to the first embodiment. As shown in Fig. 2, an example of the equipment 20 used in the equipment user support system 1 is worn on the wearer's knee. The equipment 20 includes an upper leg worn part 21, a lower leg worn part 22, and a link mechanism 23. The upper leg worn part 21 is worn on the wearer's thigh, and includes a sensor that detects an inclination of the thigh. The lower leg worn part 22 is worn on the wearer's lower leg, and includes a sensor that detects an inclination of the lower leg. In the equipment, a relative angle between the upper leg worn part 21 and the lower leg worn part 22 is calculated. Further, according to one example, when the relative angle is obtained for one of the upper leg worn part 21 and the lower leg worn part 22, the relative angle for the other one of the upper leg worn part 21 and the lower leg worn part 22 is the obtained relative angle for the one of the upper leg worn part 21 and the lower leg worn part 22 with a negative sign. It is thus not necessary to always calculate a relative angle of both wearing parts. The relative angle can also be calculated by measuring only an angle of the lower leg worn part 22 with respect to the ground. The link mechanism 23 includes, for example, a link mechanism whose rotational torque is controlled according to the angle of the knee joint. Then, the link mechanism 23 and the upper thigh worn part 21 and the lower thigh worn part 22 are connected by a hard member.

The equipment 20 assists the wearer's walking motion by controlling the rotational torque of the link mechanism to prevent the wearer from giving out his/her knee. The sensor data generated by the equipment 20 includes at least data acquisition date and time, an assist frequency of the equipment 20, a knee joint angle of the wearer, and a shin inclination angle of the wearer.

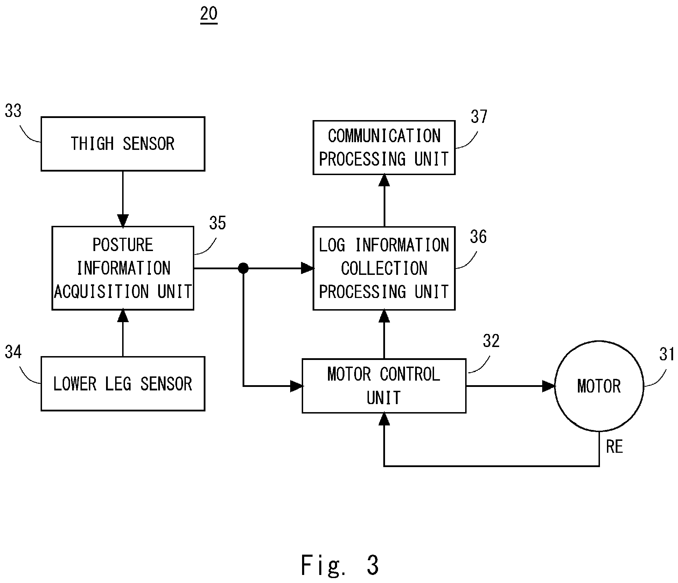

An example of a specific internal configuration of the equipment 20 will be described. Fig. 3 is a block diagram showing an internal configuration of the equipment according to the first embodiment. As shown in Fig. 3, the equipment 20 includes a motor 31, a motor control unit 32, a thigh sensor 33, a lower leg sensor 34, a posture information acquisition unit 35, a log information collection processing unit 36, and a communication processing unit 37. The motor 31 is provided in the link mechanism of the equipment 20, and generates torque to prevent the wearer from giving out his/her knee. The motor control unit 32 is a control unit that controls rotation of the motor 31. Rotation angle information RE indicating a rotation position of the motor is output from the motor 31, and the motor control unit 32 controls the rotation of the motor 31 based on the rotation angle information RE and posture information, which will described later. Further, the motor control unit 32 outputs the rotation angle information and the like used for controlling the motor 31 to the log information collection processing unit 36.

The thigh sensor 33 is a sensor provided in the upper thigh worn part 21 and detects position information indicating an inclination of the thigh and a relative position of the thigh with respect to the lower leg. The lower leg sensor 34 is a sensor provided in the lower leg worn part 22, and detects an inclination of the lower leg and position information indicating the relative position of the lower leg with respect to the thigh. The posture information acquisition unit 35 collects information output by the thigh sensor 33 and the lower leg sensor 34, and outputs the information to the log information collection processing unit 36. The log information collection processing unit 36 generates the sensor data based on the information acquired from the motor control unit 32 and the posture information acquisition unit 35, and transmits the generated sensor data to the equipment data providing system 10 via the communication processing unit 37. Note that the sensor data includes at least the data acquisition date and time, the assist frequency of the equipment 20, the knee joint angle of the wearer, and the shin inclination angle of the wearer.

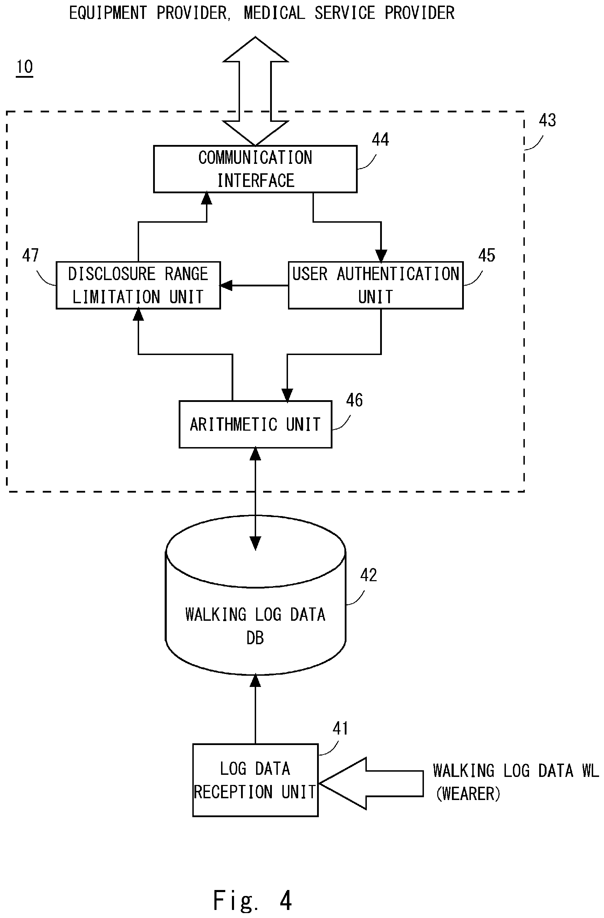

Next, a configuration of the equipment data providing system 10 will be described in detail. Fig. 4 is a block diagram of the equipment data providing system 10 according to the first embodiment. As shown in Fig. 4, the equipment data providing system 10 includes a log data reception unit 41, a walking log data database 42, and a transmission information management unit 43. The log data reception unit 41 receives the sensor data from the equipment 20 and stores it in the walking log data database 42. Note that the log data reception unit 41 selectively receives the sensor data from the equipment 20 without performing user authentication such as login processing.

The walking log data database 42 manages the walking log data in association with the adjustment information of the equipment 20 provided by the equipment provider. The transmission information management unit 43 transmits, in response to an inquiry request from outside, information managed in the walking log data database 42 to the inquirer who made the inquiry request. Specifically, the transmission information management unit 43 includes a communication interface 44, a user authentication unit 45, an arithmetic unit 46, and a disclosure range limitation unit 47.

The communication interface 44 is, for example, an interface for communicating with the medical service provider or the equipment provider via a public line. The user authentication unit 45 recognizes an attribute of a sender who sent a data registration request or the inquiry request given via the communication interface 44, and determines whether to permit login to the equipment data providing system 10. Further, the user authentication unit 45 transmits the attribute of the authenticated user and a request issued by a person who accessed to the arithmetic unit 46 and the disclosure range limitation unit 47. The arithmetic unit 46 operates the data in the walking log data database 42 based on the request given via the user authentication unit 45, and registers the adjustment information of the equipment in the walking log data database 42 or reads the data stored in the walking log data database 42. The data read by the arithmetic unit 46 is returned to the request source which has issued the request via the disclosure range limitation unit 47 and the communication interface 44.

Here, the arithmetic unit 46 may transmit, as transmission data, data acquired by applying predetermined statistical processing to the walking log data. The statistical processing for the walking log data can be performed not only by the arithmetic unit 46 but also by a terminal owned by the medical service provider or the equipment provider. The disclosure range limitation unit 47 limits the range of information to be transmitted according to the attribute of the inquirer. For example, the disclosure range limitation unit 47 does not limit the information to be disclosed to the medical service provider. However, for the equipment provider, the disclosure range limitation unit 47 sets an attribute of the equipment according to a contract type, and limits a range of information to be transmitted according to the set attribute.

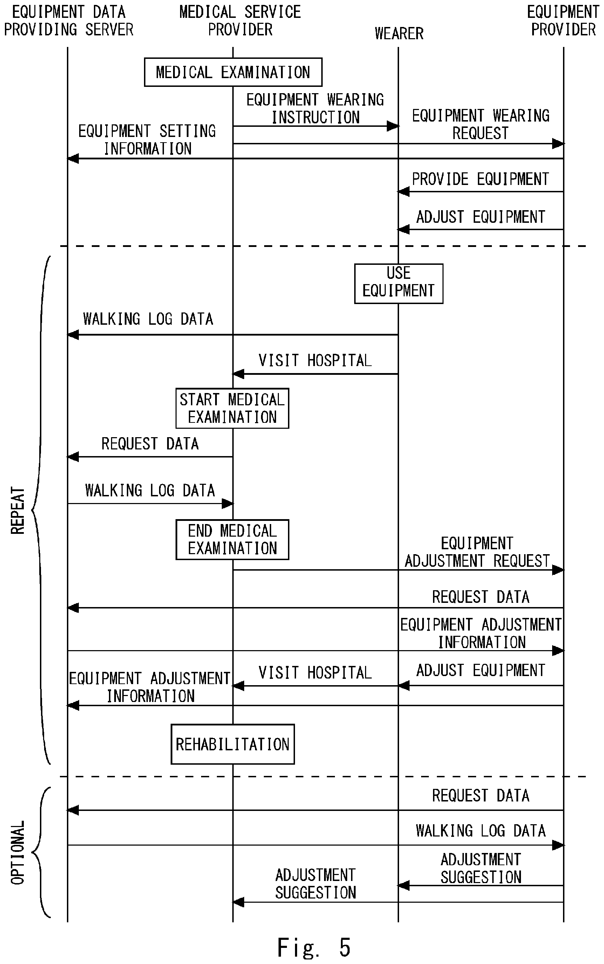

Next, a usage mode of the equipment user support system 1 will be described. Fig. 5 is a sequence diagram showing a usage mode of the equipment user support system according to the first embodiment. As shown in Fig. 5, in the equipment user support system 1, first, a medical examination is performed in the medical service provider, and the medical service provider issues an equipment wearing instruction to the wearer. Further, the medical service provider issues a request for providing equipment to be provided to the wearer to the equipment provider. When the request for providing equipment is issued, the medical service provider informs the equipment provider of information about at least one of a type of the equipment and an adjustment value of the equipment. When the adjustment value of the equipment is not informed to the equipment provider, the equipment provider sets an appropriate adjustment value.

The equipment provider which received the request registers the setting information of the equipment to be provided to the wearer in the equipment data providing system 10, and provides the adjusted equipment to the wearer. The wearer then undergoes walking training using the equipment. When the wearer undergoes walking training outside the medical service provider, the sensor data of the equipment 20 is transmitted to the equipment data providing system 10.

The wearer is examined regularly or irregularly at the medical service provider. When the wearer visits the medical service provider for the re-examination, the medical service provider requests data to the equipment data providing system 10, and refers to the history of the walking training undergone outside the medical service provider to determine the progress of the walking training. When it is determined that the equipment needs to be readjusted such as when walking training is progressing smoothly or when there is a delay in walking training, an equipment adjustment request is sent from the medical service provider to the equipment provider.

The equipment provider, which has received the equipment adjustment request, requests data to the equipment data providing system 10, acquires equipment adjustment information registered last time, and readjusts the equipment while referring to the acquired equipment adjustment information. At this time, the equipment provider re-registers the equipment adjustment information after readjustment in the equipment data providing system 10. The wearer wearing the readjusted equipment undergoes rehabilitation at the rehabilitation center 13 or the like. The processing from the start of use of the equipment to rehabilitation is repeated.

The equipment data providing system 10 provides an additional service. The additional service is to disclose the walking log data not only to the medical service provider but also to the equipment provider. An example of this additional service is optional processing shown in Fig. 5. This additional service is implemented by, for example, concluding a separate contract such as a fee-based contract between an administrator of the equipment data providing system 10 and the equipment provider. In the equipment data providing system 10, the attribute corresponding to the contract type is set for the equipment provider, and the equipment data providing system 10 limits the range of information to be disclosed to the equipment provider based on this setting.

In the optional additional service, the equipment provider may issue a data request to the equipment data providing system 10 to refer to the walking log data. The equipment provider can provide a service such as an adjustment suggestion or a visit suggestion to a wearer who visits a medical service provider less frequently based on the obtained walking log data. The equipment provider may also make a readjustment suggestion for the equipment to the medical service provider based on the acquired walking log data.

As described so far, by using the equipment data providing system 10 according to the first embodiment, it is possible for the medical service provider to determine the state of the walking training undergone by the wearer in a remote place away from the medical service provider based on objective data without using information with low accuracy such as information obtained by listening to the wearer. In other words, by using the equipment data providing system 10 according to the first embodiment, it is possible to improve the accuracy of determining whether the wearer's recovery level is caused by insufficient walking training of the wearer or by incompatibility of the equipment. Thus, by using the equipment data providing system 10 according to the first embodiment, it is possible to improve the diagnostic accuracy in the medical service provider and to lead to early recovery of the wearer's walking ability.

Further, by using the equipment data providing system 10 according to the first embodiment, it is possible to easily and reliably exchange information between the medical service provider and the equipment provider. Then, the cooperation between the medical service provider and the equipment provider is strengthened, and the support for the wearer can also be strengthened.

Furthermore, by using the equipment data providing system 10 according to the first embodiment, the medical service provider or the equipment provider can understand the state in which walking training at home is not undergone so often. Thus, it is possible for the medical service provider or the equipment provider to make a suggestion for readjustment of the equipment or a suggestion for improving motivation when training is not undergone so often.

A program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. An equipment data providing system comprising:
a database (42) configured to manage sensor data and adjustment information, and equipment (20) or a wearer (11) in association with each other, the sensor data being measured by a sensor of the equipment (20) worn by the wearer (11) for walking training, and the adjustment information indicating an adjustment state of the equipment (20); and
a transmission information management unit (43) configured to transmit information managed in the database (42) in response to an inquiry request from outside to an inquirer who makes the inquiry request.

2. The equipment data providing system according to Claim 1, wherein the adjustment information is input by a maintenance service provider for adjusting the equipment (20).

3. The equipment data providing system according to Claim 1 or 2, wherein the sensor data is stored in the database each time the wearer (11) undergoes walking training in which the wearer (11) wears the equipment (20).

4. The equipment data providing system according to any one of Claims 1 to 3, wherein
the transmission information management unit is configured to limit a range of information to be transmitted according to an attribute of the inquirer.

5. The equipment data providing system according to any one of Claims 1 to 4, wherein
when the inquirer is an equipment (20) provider which provides the equipment, the attribute is set according to a contract type, and the range of the information to be transmitted by the transmission information management unit is limited according to the set attribute.

6. The equipment data providing system according to any one of Claims 1 to 5, wherein
the transmission information management unit comprises an arithmetic unit configured to apply predetermined statistical processing to the sensor data.

7. The equipment data providing system according to any one of Claims 1 to 6, wherein
the sensor data includes at least data acquisition date and time, a frequency with which the equipment (20) assists, a knee joint angle of the wearer (11), and a shin inclination angle of the wearer as information.
